# EUROPEAN PATENT APPLICATION

(11) **EP 2 815 790 A1**
(43) Date of publication of application: **24.12.2014**
(21) Application number: 13172358.7
(22) Date of filing: 17.06.2013
(51) Int. Cl.: A61P 31/04, A61K 36/886, A61K 36/9068, A61K 36/185, A61K 36/45, A61K 36/53, A61K 36/537, A61K 36/54, A61P 31/10, A61K 31/7004

(54) **Composition for topical use in prevention and treatment of bacterial and fungal infections of skin and mucosa**

(71) Applicant: Hestia Investments, Tortola (VG)
(72) Inventor: Malkmus, Marcus, 98000 Monaco (MC); Kortschak, Kathrin, 8741 Baierdorf (AT)
(74) Representative: Gulde & Partner

(57) **Abstract**

The invention relates to a composition for topical use in prevention and treatment of bacterial and fungal infections of skin and mucosa comprising cranberry concentrate, urea, L-arginine, a buffer ensuring a pH value of the composition of 3.0 to 4.0, water and, optionally, additional active and/or auxiliary substances.

## Description

The present invention relates to an alcohol-free composition for local use in prevention and treatment of bacterial and fungal infections of skin and mucosa, especially of infections of skin and mucosa of female and male sexual organs such as e.g. vaginal infections, infectious balanitis, balanoposthitis and/or testicular infections. But the composition of the invention is also useful for prevention and treatment of bladder infection, urinary tract infections and skin infections such as e.g. athlete's foot. The composition is especially useful in restoring and stabilising a naturally balanced vaginal flora.

According to the US Center of Disease Control 29% of women aged from 14 to 49 suffer from bacterial vaginosis (BV) every year. Similarly 70-75% of women have been found to suffer from vulvovaginal candidaisis (VVC) at least once in their life or more. Both vaginal infections together are among the most frequent causes of female vaginal discomfort and disease. Both types of infections, bacterial and fungal, have been shown to significantly increase susceptibility to STDs (Sexually Transmitted Diseases), such as herpes simplex virus, human papilloma virus, HIV, clamydia and gonorrhoea. BV and VVC may also lead to complications of pregnancy and heighten the risk of preterm delivery. Although the exact causative mechanisms for BV and VVC are still little understood, it is clear that women with a sub-optimally balanced vaginal flora are more prone to these diseases.

Research is still underway trying to define a "balanced vaginal flora". So far this has primarily been defined by a surplus of H₂O₂ producing Lactobacillae combined with a vaginal pH of 4.5. However, more recent studies have demonstrated that some groups of Caucasian and Black women with healthy vaginal flora / no symptoms of BV or VVC may have a complete absence of Lactobacillae in their vaginal flora. Furthermore, the use of probiotics - be it as oral supplements or as active ingredients in vaginal suppositories / gels - has yielded differing results. It is therefore unclear, whether most commercially available products aimed at restoring or supporting a balanced and healthy vaginal flora are targeting the right mechanism or whether they only address one, potentially secondary aspect thereof.

Examples of products that focus on Lactobacillae as the main causative agent for a balanced vaginal flora are Gynophilus^{®} by Laboratoire Lycocentre, Vagi-C^{®} fem by Taurus Pharma and RepHresh^{®} Pro-B by Lil' Drug Stores.

Other products, such as Kolorex® Intimate Care by Forest Herbs Research Ltd., Deumavan® by Kaymogyn Gmbh and Vionell® Intim Salbe by Combe Pharma contain Tocopheryl acetate or other antioxidants which supposedly help restore and protect both mucosal and skin surfaces, but in a vaginal environment risk to support the proliferation of anaerobic bacteria, such as clostridium, bacteroides, peptococci and gardnerella vaginalis.

Another ingredient commonly found in commercially available products (e.g. Evaviril® by Alpenland Medica Gmbh, Vagisan® Feuchtcreme by Dr. Wolff Arzneimittel) aimed at supporting a balanced and healthy vaginal flora is alcohol which is a known skin irritant and is detrimental to vaginal mucosa.

Other products which are presently used to treat vaginal, bladder or urinary tract infections are based on antibiotics.

It was the problem of the present invention to provide an alternative, alcohol-free composition for topical use in prevention and treatment of skin and mucosal infections, especially of female and male sexual organs, which composition is not based on antibiotics and Lactobacillae. With regard to vaginal infections the composition should be able to restore and stabilise the vaginal flora's natural acidity between pH 3.0 and 4.5 over a prolonged period of time, but at least for 3 hours. The composition should be fully GRAS (generally regarded as safe) and its major components must be food-grade to allow for potential applications as a lubricant. The composition should also be fully latex condom compatible/safe and may have a lubrifying effect.

It was found by the inventors of the present application that this problem can be solved with a cosmetic composition which comprises cranberry concentrate, urea, L-arginine, a buffer ensuring a pH value of the composition of 3.0 to 4.0, water and, optionally, additional active and/or auxiliary substances. The principle of the composition is not based on antioxidative properties, that means antioxidative agents are avoided and others than those which are contained in the included plant extracts are not added to the composition not to support the proliferation of anaerobic bacteria or fungi. There is only a small amount of antioxidative compounds included which results from the cranberry concentrate and from the plant extracts which are optionally contained in the composition. On the contrary, without being wished to be bound by theory according to the present invention the use of oxidative actives seems to be essential to support the innate immune system of the sexual organs, the bladder and the urinary tract. Therefore, oxidative actives such as L- arginin are added with the aim to support the production and release of nitric oxide (NO), a major part of the local immunity.

In a preferred embodiment of the invention the cranberry concentrate (INCI name: Vaccinium Macrocarpon fruit extract) is contained from 2 to 8 wt% related to the total weight of the composition, preferably from 2.5 to 7 wt%. The extract is obtainable by extraction of the cranberry fruits with alcohol, e.g. glycerine or propylene glycol, with alcohol-water mixtures or by CO₂ extraction. A commercially available product which can be used according to the present invention is cranberry concentrate produced by Western American Foods, US.

Urea is contained in the composition of the invention preferably from 0.5 to 3 wt%, more preferred from 1.0 to 1.8 wt%, related to the total weight of the composition. In a preferred embodiment of the invention L-arginin is contained in the composition from 1 to 3 wt%, more preferred from 1.0 to 2 wt%, related to the total weight of the composition. Optionally, and additionally to L-arginine, the composition of the invention may comprise copper chloride or copper chloride dihydrate, which is a cofactor for arginase. Preferably it is contained from 0.02 to 0.08 wt% related to the total weight of the composition of the invention.

Using a buffer an acidic pH of the composition which supports antimicrobial and anti-pathogenic activity is adjusted which is from 3.0 to 4.0, preferably from 3.2 to 3.7, more preferred from 3.4 to 3.6. In a preferred embodiment of the invention the buffer comprises an alkali or alkaline earth salt of an α-hydroxy acid and an inorganic acid selected from the group comprising phosphoric, sulphuric, hydrochloric and nitric acid, preferably phosphoric acid. The alkali or alkaline earth salt is preferably a sodium, potassium, magnesium or calcium salt, more preferred a calcium salt. The α-hydroxy acid which forms the alkali or alkaline earth salt is preferably selected from the group comprising citric acid, tartaric acid, malic acid and/or oxalic acid, wherein citric acid is preferred. In an especially preferred embodiment of the invention the buffer is based on calcium citrate and phosphoric acid.

In a preferred embodiment of the invention the composition can comprise one or more plant extracts selected from the group consisting of rosemary leaf extract, cinnamon extract, hamamelis extract, ginger extract and sage extract. Each of these extracts may be contained in the composition from 0.1 to 1.0 wt% related to the total weight of the composition, preferably from 0.1 to 0.8 wt%. In an especially preferred embodiment of the invention the composition comprises all four extracts, preferably each of them with 0.1 to 0.8 wt%.

Rosemary leaf extract (INCI name: Glycerin (and) water (and) Rosmarinus officinalis leaf extract) is obtainable by extraction of the leafs with glycerine and water or by CO₂ extraction. Commercially available products which can be used according to the present invention are e.g. Rosemary Antioxidant CO₂ by Eden Botanicals, US, Rosemary Antioxidant by Aromantic, UK, Rosemary CO₂-se-plus Extract (type No. 027.021) by Flavex Naturextrakte, Germany, Rosemary Leaf Extract by Making Cosmetics, US.

Cinnamon extract (INCI name: Cinnamomum Zeylanicum Bark Extract) can be obtained by extraction with an alcohol or an alcohol-water mixture or by CO₂ extraction. Commercially available products are e.g. Cinnamon Bark CO₂ by Eden Botanicals, US, Cinnamon Bark Ceylanicum CO₂-se extract (type No. 034.001) by Flavex Naturextrakte, Germany, Cinnamon Extract by Making Cosmetics, US, Cinnamon Bark Essential Oil (Ceylon) by New Directions Aromatics, Australia.

Hamamelis extract (INCI name: Hamamelis Virginiana Twigs and/or Bark Extract) is obtainable by extraction of the bark and/or twigs with propylene or butylene glycol or their mixture with water. Commercially available products which can be used according to the present invention are e.g. Hamamelisdistillat by Jean Pütz, Germany, Hamamelis Virginiana Bark Extract by Wellgreen Technology Company, China. Sage extract (INCI name: Salvia Officinalis Leaf Extract) can be obtained by extraction of the leafs with butylene glycol or a butylene glycol - water mixture or by CO₂ extraction. Commercially available products are e.g. Sage by Eden Botanicals, US, Sage Officinalis CO₂-se extract (type No. 063.001) by Flavex Naturextrakte, Germany, Sage Extract by Making Cosmetics, US.

Ginger extract (INCI name: Zingiber Officinale Root Extract) is obtainable by extraction of the roots with propylene or butylene glycol or their mixture with water or by CO₂ extraction. Commercially available products which can be used according to the present invention are e.g. Ginger CO₂ by Eden Botanicals, US, CO₂ Extracted Ginger Essential Oil by Nanning Innovative Pharmaceutical Technology Company, China, Ginger CO₂-to Extract (type No. 014.002) by Flavex Naturextrakte, Germany, Ginger Extract by Making Cosmetics, US.

In a preferred embodiment of the invention the composition can comprise aloe vera gel (INCI name: Aloe Barbadensis Leaf Juice), preferably from 1 to 12 wt% related to the total weight of the composition, more preferred from 2 to 8 wt%. A commercially available product which can be used according to the invention is Aloe Vera Gel Concentrate, 10x Food Grade (technical/trade name) which is available by Concentrated Aloe Corporation, Ormond Beach, US.

According to another preferred embodiment of the invention the composition can comprise fucose, preferably L-fucose, and/or fucosyllactose which may be 2' - and/or 3'-fucosyllactose. Each of these components may be contained in the composition from 0.5 to 4 wt% related to the total weight of the composition, preferably from 0.5 to 2 wt%. In an especially preferred embodiment of the invention fucose, especially L-fucose, and fucosyllactose, especially 2'-fucosyllactose, may be contained in the composition, each of them preferably from 0.5 to 2 wt%.

It is an especially preferred embodiment that the composition of the invention comprises as active components cranberry concentrate, urea, L-arginin, fucose and/or fucosyllactose and the described buffer.

In another especially preferred embodiment the composition of the invention comprises as active components cranberry concentrate, urea, L-arginin, the described buffer and rosemary leaf extract, cinnamon extract, hamamelis extract, ginger extract, sage extract and aloe vera gel.

In a preferred embodiment of the invention the compositions of the invention are solutions, lotions, gels or creams.

According to the invention the compositions can optionally comprise known and in cosmetics and galenics widely used auxiliary substances which are known for the formulation of solutions, gels or creams. The contained auxiliary substances may be selected from the group comprising one or more of moisturizing substances, matrix-forming agents, oils, emulsifiers, preservatives, osmolarity regulating substances, fragrances, skin conditioning agents, and mixtures thereof.

The compositions of the invention optionally comprise a matrix forming agent selected from the group consisting of natural gums such as e.g. carrageenan, xanthan gum, guar gum, starches, pectins, agar-agar, alginates, gelatine, or carboxymethyl cellulose. Preferably natural gums are contained, especially preferred is xanthan gum. The matrix-forming agent is preferably contained from 0.8 to 2 wt% related to the total weight of the composition.

Preferred moisturizers which may be used in the composition of the invention are glycerine, propylene glycol, butylene glycol or their mixtures.

Preservatives which may be contained in the compositions of the invention comprise potassium sorbate, phenoxyethanol, sodium benzoate or their mixtures.

The compositions of the invention may be formulated as O/W or W/O emulsion. Especially suitable oils which may be used for the invention are, for example, mineral oils, hydrogenated polyisobutene, polyisoprene, squalane, calendula oil, jojoba oil, avocado oil, macadamia nut oil, castor oil, coconut oil, cotton seed oil, olive oil, safflower seed oil, sesame seed oil, soybean oil, sunflower seed oil, and mixtures thereof. Castor oil is especially preferred.

The emulsifiers which can be used in the present invention are well known by the skilled person. The emulsifiers are included in the composition of the present invention preferably in a range of 0.4 to 2 wt% related to the total weight of the composition. According to the invention especially preferred emulsifiers are lecithin, esters of C₁₂-C₂₂ fatty acids and glycerin, polyglycerin, polyalkylene glycols, sugar alcohols (e.g. sorbite), and mixtures thereof. Lecithin is especially preferred.

In a preferred embodiment of the invention the composition can comprise fragrances to improve the odor of the product. Preferably natural fragrances, such as plant or fruit extracts are used.

The composition may optionally contain sodium chloride as osmolarity regulating substance which also helps to rehydrate the mucosa.

The compositions of the present invention are useful and effective for topical application in prevention and treatment of bacterial and fungal infections of skin and mucosa, especially of skin and mucosa within and/or around female and male sexual organs and within urinary tract and bladder. The compositions are easy to apply as solution, gel, cream or similar formulations. They can be applied manually or through an applicator, which is especially useful in the case of application to the urinary tract or bladder.

The vaginal infections which can be prevented or treated with the compositions of the present invention preferably comprise bacterial vaginosis, bacterial vaginitis and/or candidiasis. The urinary tract infections which may be prevented or treated with the compositions of the present invention preferably comprise urethral infection and/or urethritis and the bladder infection which may be prevented or treated with the compositions is preferably cystitis.

The compositions can also be used as daily vaginal care products. They can be used once or more daily, as well as post urination to specifically protect the vaginal flora from alkaline urinary pH, post oral and/or coital sexual activity to restore functional vaginal pH and pre oral and/or coital sexual activity to reduce sexual activity induced alkalinity.

Other applications that have been identified through incidental use include daily penile and scrotum care where the current compositions act as antifungal and antibacterial agents helping to restore and protect the local flora and have proven effective against testicular irritations and infections, infectious balanitis and/or infectious balanoposthitis.

The compositions have also shown activity against athlete's foot and similar fungal infections of the skin that cause scaling, flaking and itching of affected areas.

The compositions themselves which comprise cranberry concentrate, urea, L-arginine, a buffer ensuring a pH value of the composition of 3.0 to 4.0, water and, optionally, additional active and/or auxiliary substances are also objects of the present invention.

The compositions of the present invention are prepared in a manner well known in pharmaceutical or cosmetic industries. Details of the preparation are given in the examples.

The following examples are offered to illustrate the compositions of the present invention and their preparation. They are not intended to be limiting in any respect.

### Example 1 : Gels

The compositions of the invention are prepared as gels in the following way:

All liquids are poured into one beaker (water, castor oil, aloe vera, rosemary cinnamon, hamamelis, sage, ginger, cranberry) and all solids into another beaker (calcium citrate, urea, xanthan, L-Arginin, copper chloride, lecithin, fucose, fucosyllactose). The beaker with liquids is then poured into mixing vessel and stirred.

Solids are gradually added and gently stirred for about 3- 5 min. Phosphoric acid is added mixed and titrated till the composition reaches a pH of 3.4 to 3.6.

| Ingredient | % | % range |
|---|---|---|
| Water | 81% | 70-85% |
| Castor Oil | 4.3% | 3-7% |
| Aloe Vera | 2% | 2-8% |
| Rosemary | 2% | 2-6% |
| Cinnamon | 0.1% | 0.1-0.8% |
| Hamamelis | 0.1% | 0.1-0.8% |
| Sage | 0.1% | 0.1-0.8% |
| Ginger | 0.1% | 0.1-0.8% |
| Cranberry Concentrate | 2.7% | 2.5-7% |
| Calcium Citrate | 1% | 1-2% |
| Urea | 1% | 0.5-3% |
| Xanthan | 1% | 0.8-2% |
| L-Arginin | 1% | 1-3% |
| Copper Chloride | 0.04% | 0.02-0.08% |
| Lecithin | 0.5% | 0.4-2% |
| Fucose | 0.8% | 0.5-2% |
| Fucosyllactose | 0.8% | 0.5-2% |
| Phosphoric Acid | Titrate to pH 3.5 | pH 3.4 - 3.6 |

### Results:

A consumer study of 10 patients was conducted based on sample formulation of Example 1. The patients were female, aged 20 to 36, and had a history of recurrent bacterial vaginosis (> once a month). All patients were assigned to apply the formulation twice daily (every morning, post urination and every evening post urination) over a period of 6 months. 10% of patients (1) reported minor adverse events (slight burning sensation). 80% of patients reported no occurrence of bacterial vaginosis during the treatment period. 20% of patients reported a single occurrence of bacterial vaginosis during the treatment period. Overall the incidence of bacterial vaginosis was reduced by 90%.

### Additional findings:

Volunteer Y (male, age 43) who signed up to the trial was suffering from Athlete's foot on both feet. The volunteer applied the formulation of Example 1 topically to both feet. The formulation was applied 3 times a day for 3 days. The infection had successfully cleared by day 3 and no recurrences were observed over the following 4 weeks.

Volunteer Z (male, age 21) had been suffering from severe balanitis (redness, itching, persistent smell) for weeks. Prior treatment efforts had failed. The volunteer signed up to the trial to apply the formulation of Example 1 topically to affected body parts twice a day. 4 days into the treatment the smell had disappeared and the redness was reduced to very minor levels. By day 7 the balanitis had disappeared.

Volunteer X (female, age 26) was suffering from urinary tract infections on a regular basis (> once a month). These urinary tract infections historically led to subsequent Bacterial Vaginosis. The volunteer chose to test the formulation of Example 1 in a two months trial. The person applied the formulation in and around vagina and urinary tract as soon as urinary tract symptoms (painful urination) occurred. The test showed that a cross infection between urinary tract and vagina could be prevented and urinary tract related symptoms subsided within several minutes.

## Claims

1. A composition for topical use in prevention and treatment of bacterial and fungal infections of skin and mucosa comprising cranberry concentrate, urea, L-arginine, a buffer ensuring a pH value of the composition of 3.0 to 4.0, water and, optionally, additional active and/or auxiliary substances.

2. Composition according to claim 1 comprising one or more plant extracts selected from the group consisting of rosemary leaf extract, cinnamon extract, hamamelis extract, ginger extract and sage extract and/or aloe vera gel.

3. Composition according to claim 1 or 2 comprising fucose and/or fucosyllactose.

4. Composition according to any one of claims 1 to 3, wherein, independently from each other, the cranberry concentrate is contained from 2 to 8 wt%, urea is contained from 0.5 to 3 wt% and L-arginin is contained from 1 to 3 wt%, the percentages being related to the total weight of the composition.

5. Composition according to any one of claims 1 to 4, wherein each of the one or more plant extracts selected from the group consisting of rosemary leaf extract, cinnamon extract, hamamelis extract, ginger extract, sage extract is contained in the composition from 0.1 to 1.0 wt%, the percentages being related to the total weight of the composition.

6. Composition according to any one of claims 1 to 5, wherein aloe vera gel is contained in the composition from 1 to 12 wt%, the percentages being related to the total weight of the composition.

7. Composition according to any one of claims 1 to 6, wherein each of fucose and/or fucosyllactose is contained in the composition from 0.5 to 4.0 wt%, the percentages being related to the total weight of the composition.

8. Composition according to any one of claims 1 to 7 comprising a matrix-forming agent, preferably from 0.8 to 2 wt%, the percentages being related to the total weight of the composition.

9. Composition according to any one of claims 1 to 8, wherein the buffer comprises an alkali or alkaline earth salt of an α-hydroxy acid and an inorganic acid selected from the group comprising phosphoric, sulphuric, hydrochloric and nitric acid.

10. Composition according to any one of claims 1 to 9 for use in prevention and treatment of bacterial and fungal infections of skin and mucosa of female and male sexual organs.

11. Composition according to claim 10 for use in prevention and treatment of vaginal infections, preferably of bacterial vaginosis, bacterial vaginitis and/or candidiasis.

12. Composition according to claim 10 for use in prevention and treatment of infectious balanitis, infectious balanoposthitis and/or testicular infections.

13. Composition according to any one of claims 1 to 9 for use in prevention and treatment of urinary tract infection, preferably of urethral infection and/or urethritis.

14. Composition according to any one of claims 1 to 9 for use in prevention and treatment of bladder infection, preferably cystitis.

15. A composition comprising cranberry concentrate, urea, L-arginine, a buffer ensuring a pH value of the composition of 3.0 to 4.0, water and, optionally, additional active and/or auxiliary substances.
